(19) Europäisches Patentamt
European Patent Office
Office européen des brevets

(11) Veröffentlichungsnummer: **0 081 790**
**A1**

(12) # EUROPÄISCHE PATENTANMELDUNG

(21) Anmeldenummer: **82111286.9**

(22) Anmeldetag: **06.12.82**

(51) Int. Cl.³: **C 07 C 87/62,** C 07 C 93/04, A 61 K 7/13

(30) Priorität: **14.12.81 DE 3149458**

(43) Veröffentlichungstag der Anmeldung: **22.06.83 Patentblatt 83/25**

(84) Benannte Vertragsstaaten: **AT BE CH DE FR GB IT LI LU NL SE**

(71) Anmelder: **Henkel Kommanditgesellschaft auf Aktien, Postfach 1100 Henkelstrasse 67, D-4000 Düsseldorf 1 (DE)**

(72) Erfinder: **Rose, David, Dr., Holbeinweg 7, D-4010 Hilden (DE)**
Erfinder: **Maak, Norbert, Dr., Liebigstrasse 18, D-4040 Neuss (DE)**

(54) **Neue p-Phenylendiamine, deren Herstellung und Verwendung.**

(57) P-Phenylendiamine der allgemeinen Formel

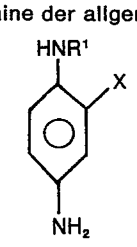

in der $R^1$ einen Rest $-(CHR^2-CHR^2-O)_n-H$ oder 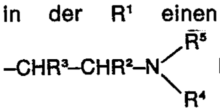 bedeutet, wobei $R^2$ und $R^5$ Wasserstoff oder Alkylgruppen mit 1–4 Kohlenstoffatomen, $R^3$ und $R^4$ Alkylgruppen mit 1–4 Kohlenstoffatomen oder n ganze Zahlen von 1–4 bedeuten und in der X für ein Fluoratom oder, wenn $R^1$ eine Aminoalkylgruppe bedeutet, für ein Wasserstoffatom steht, sind neue Entwicklersubstanzen für Oxidationshaarfärbemittel. Ihre Herstellung erfolgt durch Umsetzung von 1,2-Difluor-4-nitrobenzol mit einem Polyglycolamin bzw. durch Umsetzung von p-Fluornitrobenzol mit $N^1$,$N^1$-Dialkyl-1,2-propandiamin und katalytische Hydrierung der erhaltenen p-Nitrophenylamine.

Oxidationshaarfärbemittel mit den neuen Entwicklersubstanzen und üblichen Kupplersubstanzen zeichnen sich durch intensive, natürliche Farbtöne mit guten Echtheitseigenschaften und guter dermatologischer Verträglichkeit aus.

EP 0 081 790 A1

0081790

4000 Düsseldorf, den 10.12.1981
Henkelstraße 67

HENKEL KGaA
ZR-FE/Patente
Ba/Et

Patentanmeldung

D 6447 EP

"Neue p-Phenylendiamine, deren Herstellung und Verwendung"

Die Erfindung betrifft neue p-Phenylendiamine und Haarfärbemittel, welche diese als Entwicklerkomponente enthalten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Amino-pyrazolon-derivate, heterocyclische Hydrazone eingesetzt. Als sogenannte Kupplerkomponenten werden m-Phenylen-diaminderivate, Phenole, Naphthole, Resorcinderivate und Pyrazolone genannt.

Gute Oxidationshaarfarbstoffkomponenten müssen in erster Linie folgende Voraussetzungen erfüllen:

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen und sie sollen darüber hinaus in toxikologischer und dermatologischer Hinsicht unbedenklich sein.

...

Die üblicherweise als Entwicklersubstanzen verwendete Verbindungsklasse der substituierten bzw. unsubstituierten p-Phenylendiamine besitzt den Nachteil, daß sie bei einer Reihe von Personen Sensibilisierungen und in deren Gefolge schwere Allergien hervorruft. Die zur Vermeidung dieser dermatologischen Nachteile in neuerer Zeit vorgeschlagenen Entwicklersubstanzen können in ihren anwendungstechnischen Eigenschaften nicht immer voll befriedigen.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurden nun neue p-Phenylendiamin-Derivate gefunden, welche als Entwicklersubstanzen mit den in Oxidationshaarfarben üblichen Kuppler-Komponenten den gestellten Anforderungen in besonders hohem Maße gerecht werden. Gegenstand der Erfindung sind p-Phenylendiamine der Formel I

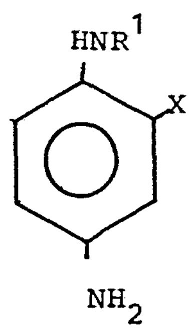

(I)

in der $R^1$ für einen Polyalkoholrest der Formel $-[CHR^2-CHR^2-O]_n-H$ oder eine Aminoalkylgruppe der Formel $-CHR^3-CHR^2-N(R^5)(R^4)$ steht, wobei $R^2$ und $R^5$ ein Wasserstoffatom

...

oder eine Alkylgruppe mit 1 - 4 Kohlenstoffatomen, $R^3$ und $R^4$, eine Alkylgruppe und 1 - 4 Kohlenstoffatomen und n eine ganze Zahl von 1 - 4 bedeutet, und in der X für ein Fluoratom oder wenn $R^1$ für eine Aminoalkylgruppe steht für ein Wasserstoffatom steht, sowie ein Verfahren zu deren Herstellung. Ein weiterer Gegenstand der Erfindung sind Haarfärbemittel, welche eine p-Phenylendiamin-Verbindung der Formel (I) als Entwicklerkomponente neben den in Oxidationshaarfarben üblichen Kupplerkomponenten und Träger- und Hilfsstoffen enthalten. Anstelle der p-Phenylendiaminderivate der Formel I können in den Haarfarben auch deren anorganische oder organische Salze eingesetzt werden.

Besonders bevorzugt als Entwicklerkomponenten werden die Derivate der Formel II und III.

...

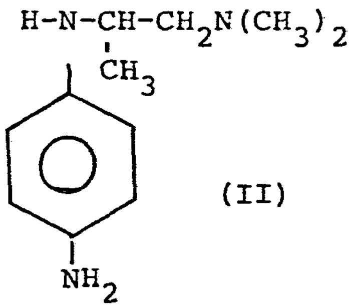

(II)

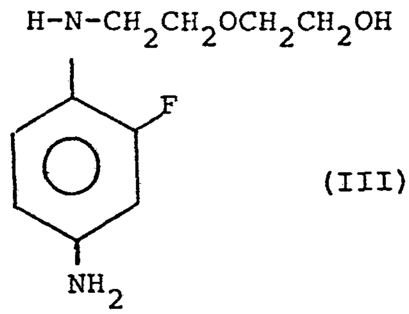

(III)

Bei ihrem Einsatz als Entwicklerkomponenten liefern die erfindungsgemäßen Verbindungen mit den im allgemeinen für die Oxidationshaarfärbung verwendeten Kupplersubstanzen die unterschiedlichsten sehr intensiven Farbtöne und stellen somit eine wesentliche Bereicherung der oxidativen Haarfärbemöglichkeiten dar. Darüber hinaus zeichnen sich die erfindungsgemäßen hydroxyalkylierten Alkylamino-p-phenylendiamine durch sehr gute Echtheitseigenschaften der damit erzielten Färbungen, durch eine gute Löslichkeit im Wasser, eine gute Lagerstabilität und toxikologische, sowie dermatologische Unbedenklichkeit aus.

Die erfindungsgemäß als Entwicklerkomponenten zu verwendenden hydroxyalkylierten Alkylamino-p-phenylendiamine können entweder als solche oder in Form ihrer Salze mit anorganischen oder organischen Säuren, wie z. B. als Chloride, Sulfate, Phosphate, Acetate, Propionate, Lactate, Citrate eingesetzt werden.

Zur Herstellung der erfindungsgemäßen 2-Fluor-N-polyetheralkohol-p-phenylendiamine wird in erster Stufe 1,2-Difluor-4-nitrobenzol mit Di- bzw. Polyglykolamid gemischt und bei erhöhter Temperatur, vorzugsweise 1oo - 14o$^{o}$ C umgesetzt und anschließend auf Eiswasser gegossen. Das sich dabei abscheidende Öl wird vorzugsweise mit Ether extrahiert, das Extraktionsmittel ab-

...

destilliert und das erhaltene N-(2-Fluor-4-aminophenyl)-Di- bzw. Polyglykolamin in einer zweiten Stufe in Gegenwart eines Hydrierkatalysators, vorzugsweise Raney-Nickel hydriert.

Zur Herstellung von N-(p-Aminophenyl)-N'-dialkyl-1,2-diaminoalkan wird in einer ersten Stufe $N^1$, $N^1$-Dialkyl-1,2-dialkandiamin in Dimethylformamid mit Kaliumcarbonat bei erhöhter Temperatur mit einer vorzugsweise äquimolaren Menge p-Fluornitrobenzol umgesetzt. Nach mehrstündiger Reaktionszeit bei 12o - 18o, vorzugsweise 15o - 16o° C, wird die Mischung auf Eiswasser gegossen, das abgeschiedene Öl extrahiert und das Extraktionsmittel abdestilliert. Das erhaltene N-(p-Nitrophenyl)-N'-dialkyl-1,2-diaminoalkan wird in einer zweiten Stufe in Gegenwart eines Hydrierkatalysators hydriert.

Die erfindungsgemäßen Entwicklerkomponenten können mit verschiedensten als Kupplerkomponenten bekannten Substanzen wie α-Naphthol, o-Kresol, m-Kresol, 2,6-Dimethylphenol, 2,5-Dimethylphenol, Brenzcatechin, Pyrogallol, 1,5- bzw. 1,7-Dihydroxynaphthalin, 5-Amino-2-methylphenol, m-Aminophenol, 2,4-Diaminoanisol, m-Toluylendiamin, Resorcin, Resorcinmonomethylether, m-Phenylendiamin, 1-Phenyl-3-methyl-pyrazolon-5, 1-Amino-3-acetylacetamino-4-nitrobenzol, 1-Amino-3-cyanacetylamino-3-nitrobenzol, 2,4-Dichlor-3-aminophenol, 1,3-Bis-(2,4-diaminophenoxy)-propan, 2-Chlorresorcin, 2-Chlor-6-methyl-3-aminophenol, 2,7-Dihydroxynaphthalin, 2-Methylresorcin, 2,4-Diaminophenol und 2,4-Dichlor-3-isopropyliden-aminophenol, zu Haarfarbstoffen umgesetzt werden.

In den erfindungsgemäßen Haarfärbemitteln werden die Entwicklerkomponenten im allgemeinen in etwa molaren

...

Mengen, bezogen auf die verwendeten Kupplersubstanzen, eingesetzt. Wenn sich auch der molare Einsatz als zweckmäßig erweist, so ist es jedoch nicht nachteilig, wenn die Entwicklerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemisch der erfindungsgemäß zu verwendenden hydroxyalkylierten Alkylamino-p-phenylendiamine als auch die Kupplersubstanz Gemische der vorstehend genannten Kupplerkomponenten darstellen.

Darüber hinaus können die erfindungsgemäßen Haarfärbemittel andere bekannte und übliche Entwicklerkomponenten, sowie auch gegebenenfalls übliche direktziehende Farbstoffe im Gemisch enthalten, falls dies zur Erzielung gewisser Farbnuancen erforderlich ist.

Die oxidative Kupplung, d. h. die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat, sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende ksometische Zubereitungen, wie Cremes, Emulsionen, Gele oder auch einfache Lösungen eingearbeitet und unmittelbar vor der Anwendung auf dem

...

Haar mit einem der genannten Oxidationsmittel versetzt. Die Konzentration derartiger färberischer Zubereitungen an Kuppler-Entwicklerkombination beträgt o,2 bis 5 Gewichtsprozent, vorzugsweise 1 - 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind z. B. Netz- oder Emulgiermittel vom anionischen oder nichtionogenen Typ, wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel, wie Pantothensäure und Cholesterin zu nennen. Die genannten Zusatzstoffe werden dabei in den für diese Zwecke üblichen Mengen eingesetzt, wie z. B. Netz- und Emulgiermittel in Konzentrationen von o,5 - 3o Gewichtsprozent und Verdickungsmittel in Konzentrationen von o,1 - 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 - 1o erfolgen. Die Anwendungstemperaturen bewegen sich dabei im Bereich von 15 bis 4o° C. Nach einer Einwirkungsdauer von ca. 3o Minuten wird das Haarfärbemittel vom zu färbenden Haar durch Spülen entfernt. Danach wird das Haar mit einem milden Shampoo nachgewaschen und getrocknet.

...

Es werden sehr intensive und natürliche Farbtöne, vorzugsweise Braun- bis Dunkelblautöne erreicht. Die Farbstoffe haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn darauf zu beschränken.

## Beispiel 1

Herstellung von N-(2-Fluor-aminophenyl)-diglykolamin-dihydrochlorid

### Stufe 1

N-(2-Fluor-4-nitrophenyl)-diglykolamin

o,o6 Mol (1o g) 1,2-Difluor-4-nitrobenzol werden mit o,13 Mol (13,7 g) Diglykolamin gemischt. Die Mischung wird auf 11o° C erhitzt und nach 1,5 Stunden auf ca. 1oo ml Eiswasser gegossen. Das abgeschiedene Öl wird mit Ether extrahiert. Nach dem Trocknen über Natriumsulfat wird der Ether abdestilliert.

Ausbeute: 16,7 g gelbes Öl
IR-Spektrum ($cm^{-1}$): 1615, 1545, 15oo, 137o, 133o, 13oo, 1195, 115o, 1125, 1o7o, 945, 88o, 8o5

### Stufe 2

N-(2-Fluor-4-aminophenyl)-diglykolamindihydrochlorid

1o g (o,o65 Mol) N-(2-Fluor-4-nitrophenyl)-diglykolamin werden in 1oo ml Ethanol in Gegenwart von Raney-Nickel (ca. 1 g) bei Zimmertemperatur und 1 atü hydriert. Nach beendeter $H_2$-Aufnahme wird vom Katalysator abfiltriert. Die Lösung wird mit Salzsäure angesäuert und zur Trockene eingeengt.

Ausbeute: 14,3 g farblose Kristalle; Fp. 146° C.

...

Beispiel 2

Herstellung von N-(p-Aminophenyl)-N'-dimethyl-1,2-diaminopropan-trihydrochlorid

Stufe 1

N-(p-Nitrophenyl)-N'-dimethyl-1,2-diaminopropan

Zu einer Suspension von 9,5 g (o,o9 Mol) $N^1,N^1$-Dimethyl-1,2-propandiamin in 2o ml Dimethylformamid und 8,3 g (o,o6 Mol) Kaliumcarbonat bei 9o° C werden 13,1 g (o,o9 Mol) p-Fluornitrobenzol zugetropft. Nach 4 Stunden bei 16o° C wird abgekühlt und auf 25o ml Eiswasser gegossen. Das abgeschiedene Öl wird mit Essigester extrahiert. Nach dem Trocknen über Natriumsulfat wird zur Trockene eingeengt.

Ausbeute: 16 g gelbes Öl.

Stufe 2

N-(p-Aminophenyl)-N'-dimethyl-1,2-propandiamintrihydrochlorid

16,2 g N-(p-Nitrophenyl)-N'-dimethyl-1,2-propandiamin werden in 2oo ml Ethanol in Gegenwart von 5 % Palladium auf Kohle bei 25° C und 1 atü hydriert. Nach beendeter $H_2$-Aufnahme wird vom Katalysator abfiltriert, mit verdünnter Salzsäure angesäuert und zur Trockene eingeengt.

Ausbeute: 16 g weiße Kristalle
IR-Spektrum ($cm^{-1}$): 163o, 161o. 157o, 151o, 148o, 1465, 132o, 126o, 119o, 113o, 1o65, 1o18, 1ooo, 98o, 92o, 82o.

...

Beispiel 3

Die nach Beispiel 1 und 2 hergestellten p-Phenylendiamine wurden in Haarfärbemittel in Form einer Cremeemulsion zu Färbetests herangezogen. Dabei wurden in eine Emulsion aus

1o Gewichtsteilen Fettalkoholen der Kettenlänge $C_{12}$-$C_{18}$
1o Gewichtsteilen Fettalkoholsulfat (Natriumsalz) Kettenlänge $C_{12}$-$C_{18}$
75 Gewichtsteilen Wasser

jeweils o,o1 Mol der erfindungsgemäßen p-Phenylendiaminderivate und die nachstehend aufgeführten Kupplersubstanzen eingearbeitet. Danach wurde der pH-Wert der Emulsion mittels Ammoniak auf 9,5 eingestellt und die Emulsion mit Wasser auf 1oo Gewichtsteile aufgefüllt. Die oxidative Kupplung wurde mit 9 %iger Wasserstoffperoxidlösung als Oxidationsmittel durchgeführt, wobei zu 1oo Gewichtsteilen der Emulsion 1o Gewichtsteile Wasserstoffperoxidlösung gegeben wurden. Die jeweilige Färbecreme mit zusätzlichem Oxidationsmittel wurde auf zu 9o % ergrautes, nicht besonders vorbehandeltes Menschenhaar aufgetragen und dort 3o Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle I zu entnehmen.

Als Kupplersubstanzen dienten folgende Verbindungen:

1. Resorcin
2. 4-Chlorresorcin
3. 1-Naphthol

...

4. 5-Amino-2-methylphenol
5. 1,3-Bis-(2,4-diaminophenoxy)-propan
6. 1-Phenyl-3-acetamido-pyrazolon-5

...

TABELLE 1

| Farbstoffkomponenten | | Nuance des gefärbten Haares |
|---|---|---|
| a) Entwickler | b) Kuppler | 9 %ige $H_2O_2$-Lösung |
| 1) N-(2-Fluor-4-aminophenyl)-Diglykolamindihydrochlorid | Resorcin | braun |
| 2) " " | 4-Chlorresorcin | braun |
| 3) " " | 1-Naphthol | grauviolett |
| 4) " " | 5-Amino-2-methylphenol | graurubin |
| 5) " " | 1,3-Bis-(2,4-diaminophenoxy)-propan | tintenblau |
| 6) N-(p-Aminophenyl)-N'-dimethyl-1,2-diaminopropantrihydrochlorid | Resorcin | braun |
| 7) " " | 1-Phenyl-3-acetamido-pyrazolon-5 | rotbraun |
| 8) " " | 1,3-Bis-(2,4-diaminophenoxy)-propan | dunkelblau |
| 9) " " | 4-Chlorresorcin | braun |
| 1o) " " | 1-Naphthol | dunkelviolett |
| 11) " " | 5-Amino-2-methylphenol | dunkelrubin |

...

Patentansprüche

1. p-Phenylendiamine der allgemeinen Formel I:

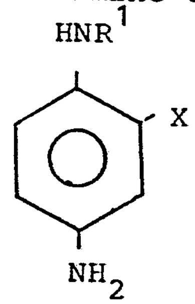

(I)

in der $R^1$ für einen Polyalkoholrest der Formel $\{CHR^2-CHR^2-O\}_n H$ oder eine Aminoalkylgruppe der Formel $-CHR^3-CHR^2-N<\begin{smallmatrix}R^5\\R^4\end{smallmatrix}$ steht, wobei $R^2$ und $R^5$ ein Wasserstoffatom oder eine Alkylgruppe mit 1 - 4 Kohlenstoffatomen, $R^3$ und $R^4$, eine Alkylgruppe und 1 - 4 Kohlenstoffatomen und n eine ganze Zahl von 1 - 4 bedeutet, und in der X für ein Fluoratom oder wenn $R^1$ für eine Aminoalkylgruppe steht für ein Wasserstoffatom steht.

2. N-(2-Fluor-4-aminophenyl)-diglykolamin.

3. N-(p-Aminophenyl)-N'-dimethyl-1,2-diaminopropan.

4. Verfahren zur Herstellung von p-Phenylendiamin-Verbindungen der allgemeinen Formel IV

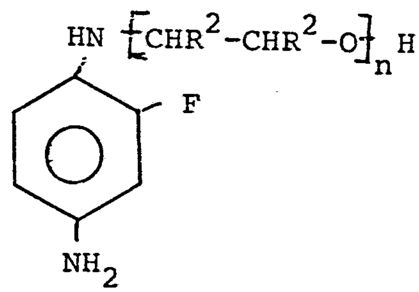

(IV)

in der $R^2$ und n die obengenannte Bedeutung haben, dadurch gekennzeichnet, daß man 1,2-Difluor-4-nitrobenzol mit einem Polyglykolamin bei erhöhter Temperatur umsetzt und das Reaktionsprodukt durch Extraktion abtrennt und in Gegenwart eines Hydrierkatalysators hydriert.

...

5. Verfahren zur Herstellung einer p-Phenylendiamin-Verbindung der Formel V

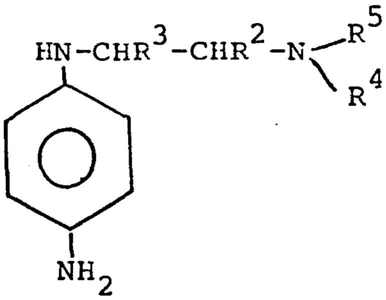

(V)

wobei $R^2$, $R^3$, $R^4$ und $R^5$ die obengenannte Bedeutung hat, dadurch gekennzeichnet, daß man $N^1$, $N^1$-Dimethyl-1,2-propandiamin in Dimethylformamid, Kaliumcarbonat und p-Fluornitrobenzol bei erhöhter Temperatur umsetzt, das Reaktionsprodukt durch Extraktion abtrennt und in Gegenwart eines Hydrierkatalysators hydriert.

6. Haarfärbemittel auf Basis von Oxidationsfarbstoffen, gekennzeichnet durch einen Gehalt an einer p-Phenylendiaminverbindung der Formel (I), sowie deren anorganischen oder organischen Salzen als Entwicklerkomponenten und den in Oxidationshaarfarben üblichen Kupplersubstanzen, Träger- und Hilfsstoffen.

7. Haarfärbemittel nach Anspruch 6, gekennzeichnet durch einen Gehalt an N-(2-Fluor-4-aminophenyl)-diglykolamin oder seinen anorganischen oder organischen Salzen als Entwicklerkomponente.

8. Haarfärbemittel nach Anspruch 6, gekennzeichnet durch einen Gehalt an N-(p-Aminophenyl)-N'-dimethyl-1,2-diaminopropan oder seinen anorganischen oder organischen Salzen als Entwicklerkomponente.

...

9. Haarfärbemittel nach Anspruch 6 bis 8, gekennzeichnet durch einen Gehalt an Entwickler-Kuppler-Kombination von o,2 bis 5 Gewichtsprozent, vorzugsweise 1 bis 3 Gewichtsprozent, bezogen auf das gesamte Mittel.

Europäisches Patentamt

# EUROPÄISCHER RECHERCHENBERICHT

0081790 Nummer der Anmeldung

EP 82 11 1286

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | GB-A-2 018 808 (L'OREAL) * Seite 3, Zeilen 36-55 * |  | C 07 C 87/62<br>C 07 C 93/04<br>A 61 K 7/13 |
| A | GB-A-1 048 790 (SCHWARZKOPF VERWALTUNG GmbH) * Beispiel VII * |  |  |
| A | GB-A-2 018 302 (L'OREAL) * Seite 16, Zeilen 5,6 * | 1,2,4-7,9 |  |
| A | JOURNAL OF MEDICINAL CHEMISTRY, Band 15, Nr. 5, 1972, Seiten 523-529, Washington D.C., USA C.J. SHARPE et al.: "Basic ethers of 2-anilinobenzothiazoles and 2-anilinobenzoxazoles as potential antidepressants" * Seite 527, rechte Spalte, Zeilen 63-65 * |  | RECHERCHIERTE SACHGEBIETE (Int. Cl. 3)<br>C 07 C 87/00<br>C 07 C 93/00 |

Der vorliegende Recherchenbericht wurde für alle Patentansprüche erstellt.

| Recherchenort | Abschlußdatum der Recherche | Prüfer |
|---|---|---|
| DEN HAAG | 17-03-1983 | PAUWELS G.R.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument